# EUROPÄISCHE PATENTSCHRIFT

(11) **EP 4 196 199 B1**
(45) Veröffentlichungstag und Bekanntmachung des Hinweises auf die Patenterteilung: **08.11.2023**
(21) Anmeldenummer: 21786791.0
(22) Anmeldetag: 27.09.2021
(51) Int. Cl.: A61M 16/04, A61M 16/06, A61M 25/02

(54) **BEFESTIGUNGSMITTEL FÜR TRACHEALKANÜLEN**
FASTENING MEANS FOR TRACHEAL CANNULAE
MOYEN DE FIXATION DE CANULES DE TRACHÉOTOMIE

(30) Priorität: 28.09.2020 DE 102020125292
(43) Veröffentlichungstag der Anmeldung: 21.06.2023
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph
(86) Internationale Anmeldenummer: PCT/EP2021/076505
(87) Internationale Veröffentlichungsnummer: WO 2022/064036

(56) Entgegenhaltungen:
- US-A1- 2006 124 133
- US-A1- 2017 281 895
- US-A1- 2018 078 727
- US-A1- 2019 381 267

## Beschreibung

Die Erfindung umfasst ein Befestigungsmittel für Trachealkanülen sowie ein Set umfassend zumindest zwei Klebeplatten und mindestens zwei mit den Klebeplatten verbindbare Bänder, wobei mittels jeweils eines Bandes und einer Klebeplatte ein erfindungsgemäßes Befestigungsmittel bildbar ist.

Aus dem Stand der Technik sind Befestigungsmittel für Trachealkanülen allgemein bekannt. So werden Trachealkanülen üblicher Weise mit einem Band, das um den Hals des Benutzers geführt ist, befestigt. Zur Fixierung der Trachealkanüle in dem Tracheostoma des Benutzers muss das Band fest am Hals anliegen. Diese Art der Befestigung wird jedoch von einigen Benutzern als unangenehm empfunden. Bei Bewegungen kann das Band auf der Hautoberfläche Reizungen auslösen, unter dem Band wird geschwitzt, das Band wird dreckig oder es liegt auf einer Wunde, beispielsweise unmittelbar nach einer Kehlkopfoperation, auf.

EP 1 477 197 B1 offenbart eine Alternative zu den aus dem Stand der Technik bekannten Bändern, wobei Klebestreifen auf die Haut des Benutzers aufgebracht werden. Auf diesen aufgebracht ist jeweils ein Haken, die in jeweils eine Ausnehmung der Flügel einer Trachealkanüle eingreifen können. Weiterhin wird eine Ausgestaltung offenbart, bei der von dem Klebestreifen ein Band mit einem Klettverschluss abgeht, das in die Halteöse fädelbar ist, wobei das Band umklappbar an sich selbst haftet, um eine Halteschlaufe zu bilden.

US 2017/281895 A1 offenbart ein Befestigungsmittel für Trachealkanülen mit einer Klebeplatte und einem Klettverschlussband.

Nachteilig an dem aus dem Stand der Technik bekannten Befestigungsmitteln für Trachealkanülen ist die mangelhafte Justierbarkeit. Tracheostoma können so tief ausgebildet sein, dass Bänder die Trachealkanüle teilweise aus dem Tracheostoma heraus ziehen oder diese zumindest anwinkeln, was als unangenehm empfunden wird. Das in EP 1477 197 B1 beschriebene Befestigungsmittel kann tiefer angesetzt werden als die Bänder Ist dieses einmal auf der Hautoberfläche aufgebracht, kann eine (Nach-)Justierung, insbesondere durch den Benutzer, selber nicht mehr vorgenommen werden. Hierzu müssten die Klebestreifen entfernt und neue auf der Haut aufgebracht werden. Eine Anpassung über den Tag an bestimmte Situationen, in jenen der Kopf des Trägers einer Trachealkanäle öfters hoch oder herunter geneigt wird, ist mit den aus dem Stand der Technik bekannten Befestigungsmitteln nicht möglich.

Aufgabe der vorliegenden Erfindung ist es, ein verbessertes Befestigungsmittel für Trachealkanülen zur Verfügung zu stellen. Insbesondere ist es Aufgabe der Erfindung, ein Befestigungsmittel zur Verfügung zu stellen, dass einfach (nach-)justiert und an eine Tragesituation, wie beispielsweise das Schauen eines Kinofilms, bei der der Kopf des Benutzers längere Zeit nach oben geneigt ist, oder bei feinmechanischen Arbeiten, bei denen der Kopf längere Zeit nach unten geneigt ist, angepasst werden kann. Weiter bevorzugt ist Aufgabe der Erfindung, eine sichere Fixierung für Trachealkanülen zu gewährleisten, die insbesondere trotz Einstellmöglichkeiten an eine Tragesituation sicher die Trachealkanüle in der gewünschten Position hält, insbesondere auch bei einer Bewegung des Benutzers.

Die Aufgabe ist erfindungsgemäß gelöst durch ein Befestigungsmittel für Trachealkanülen umfassend eine Klebeplatte mit einer ersten Oberfläche und einer zweiten Oberfläche und mindestens ein mit der Klebeplatte verbundenes Band zur Verbindung mit einem Haltemittel einer Trachealkanüle, wobei die erste Oberfläche eine Klebeschicht zum Aufkleben auf eine Hautoberfläche, insbesondere eines Benutzers beziehungsweise Trägers der Trachealkanüle, und die zweite Oberfläche ein erstes Klettverschlussteil umfasst, wobei das Band zumindest einseitig ein zweites Klettverschlussteil umfasst, das mit dem ersten Klettverschlussteil der Klebeplatte korrespondiert, wobei sich das erste Klettverschlussteil vollflächig über die erste Oberfläche der Klebeplatte erstreckt.

Weiterhin ist die Aufgabe erfindungsgemäß gelöst durch ein Set umfassend zumindest zwei Klebeplatten mit jeweils einer ersten Oberfläche und einer zweiten Oberfläche und mindestens zwei mit den Klebeplatten verbindbare Bänder, diese zur Verbindung mit einem Haltemittel einer Trachealkanüle, wobei jeweils die erste Oberfläche der Klebeplatten eine Klebeschicht zum Aufkleben auf eine Hautoberfläche und jeweils die zweite Oberfläche ein erstes Klettverschlussteil umfasst, wobei sich das erste Klettverschlussteil vollflächig über die erste Oberfläche der Klebeplatte erstreckt, wobei die Bänder jeweils zumindest einseitig ein zweites Klettverschlussteil umfassen, das mit dem ersten Klettverschlussteil zumindest einer der Klebeplatten korrespondiert, wobei mittels jeweils eines Bandes und einer Klebeplatte ein Befestigungsmittel bildbar ist.

Es wird ein Befestigungsmittel für Trachealkanülen vorgeschlagen. Das Befestigungsmittel umfasst eine Klebeplatte mit einer ersten Oberfläche und einer zweiten Oberfläche und mindestens ein mit der Klebeplatte verbundenes Band zur Verbindung mit einem Haltemittel einer Trachealkanüle. Die erste Oberfläche umfasst eine Klebeschicht zum Aufkleben auf eine Hautoberfläche und die zweite Oberfläche umfasst ein erstes Klettverschlussteil. Das Band umfasst zumindest einseitig eine zweites Klettverschlussteil, die mit dem ersten Klettverschlussteil der Klebeplatte korrespondiert, wobei sich das erste Klettverschlussteil vollflächig über die erste Oberfläche der Klebeplatte erstreckt.

Mittels der Klebeplatte kann das Haltemittel auf der Haut eines Benutzers befestigt werden. Durch die klebende Befestigung auf der Haut können insbesondere Druckstellen verhindert werden. Auch ist es möglich, die Klebeplatte exakter zu positionieren und an die Gegebenheiten des Tracheostomas und des umliegenden Bereiches anzupassen.

Die Klebeplatte ist bevorzugt flexibel ausgestaltet und ist weiter bevorzugt an eine Hautoberfläche des Benutzers anpassbar. Insbesondere ist die Klebeplatte derart biegbar beziehungsweise verformbar, dass diese sich an die Hautoberfläche des Benutzers anlegen kann. In einer Ausgestaltung umfasst die Klebeplatte ein textiles Grundmaterial. In einer weiteren Ausgestaltung umfasst die Klebeplatte ein folienartiges Grundmaterial. In einer weiteren Ausgestaltung umfasst die Klebeplatte ein Grundmaterial, welches offenporigen oder geschlossenporigen Schaumstoff umfasst. Die Klebeplatte umfasst eine erste Oberfläche und eine, der ersten Oberfläche gegenüberliegende, zweite Oberfläche. Die erste Oberfläche umfasst die Klebeschicht, die insbesondere einen Kleber aufweist, der zum Aufkleben auf die Haut des Benutzers vorgesehen ist. Durch die Klebeschicht wird insbesondere erreicht, dass das Befestigungsmittel fixiert ist und die Trachealkanüle in dem Tracheostoma ausreichend genau in Position bleibt. Erfindungsgemäß ist die Klebeschicht vollflächig auf der ersten Oberfläche aufgebracht, sie kann aber auch teilflächig aufgebracht sein. In einer alternativen Ausgestaltung ist vorgesehen, dass die Klebeschicht in Streifen aufgebracht ist, so dass insbesondere abwechselnd ein Streifen Klebeschicht und ein Oberflächenteil der ersten Oberfläche, der nicht klebend ausgestaltet ist, vorgesehen ist.

In einer weiteren Ausgestaltung ist vorgesehen, dass auf der Klebeschicht eine Schutzschicht vorgesehen ist. Die Schutzschicht ist insbesondere dazu geeignet, die Klebeschicht vor Staub und/oder Verschmutzung zu schützen, bevor das Befestigungsmittel verwendet wird. Durch die Schutzschicht wird die Haltbarkeit vorteilhafterweise verlängert, so dass das Befestigungsmittel auch noch nach längerer Lagerung verwendbar ist. In einer bevorzugten Ausgestaltung ist vorgesehen, dass die Schutzschicht eine Lasche zum Lösen der Schutzschicht von der Klebeschicht aufweist. In einer Ausgestaltung ist vorgesehen, dass die Lasche über die Klebeschicht, bevorzugt über die erste Oberfläche der Klebeplatte hinaus ragt und somit leicht gegriffen werden kann. In einer weiteren Ausgestaltung ist vorgesehen, dass die Lasche durch einen Teilschnitt der Klebeplatte, insbesondere an einer Ecke, gebildet ist. Der Teilschnitt ist insbesondere ein Schnitt durch das Material der Klebeplatte, bevorzugt durch das Grundmaterial, wobei die Schutzschicht, insbesondere nur die Schutzschicht, weiter bevorzugt nur die Schutzschicht und die Klebeschicht, nicht geschnitten ist. Bevorzugt ist hierzu eine Ecke der Klebeplatte zumindest teilweise abgetrennt. Der Benutzer kann beispielsweise an der teilgeschnitten Ecke der Klebeplatte die Schutzschicht greifen und mittels einer Scherbewegung die Schutzschicht von der Klebeschicht ablösen. Die Lasche bietet somit eine Grifffläche zum Ablösen der Schutzschicht, ohne diese hierzu mit den Fingernägeln oder sonstigen Hilfsmitteln lösen zu müssen.

Die zweite Oberfläche umfasst ein erstes Klettverschlussteil, das mit einem nachfolgend beschriebenen zweiten Klettverschlussteil eines Bandes des Befestigungsmittels zusammen wirkt. Bevorzugt bilden das erste Klettverschlussteil und das zweite Klettverschlussteil einen Haftverschluss beziehungsweise einen Klettverschluss. Insbesondere ist im Sinne der Erfindung unter einem Klettverschluss ein Fixiermittel zu verstehen, dass zwei korrespondierende, aufeinander haftende Teile aufweist. Das erste Klettverschlussteil kann Widerhaken, Schlaufen, Rundköpfe und/oder Pilzköpfe aufweisen, die bevorzugt mit Widerhaken, Schlaufen, Rundköpfen und/oder Pilzköpfen des zweiten Klettverschlussteils des Bandes korrespondieren. Das erste Klettverschlussteil und das zweite Klettverschlussteil korrespondieren miteinander, indem diese eine Haftverbindung miteinander eingehen können. So ist beispielsweise unter "korrespondieren" zu verstehen, dass das erste Klettverschlussteil und das zweite Klettverschlussteil derart unterschiedlich ausgestaltet, dass beispielsweise Widerhaken des zweiten Klettverschlussteils in Schlaufen des ersten Klettverschlussteils eingreifen. In einer Ausgestaltung ist vorgesehen, dass das erste Klettverschlussteil, das Pilzköpfe aufweist, mit dem zweiten Klettverschlussteil, das Pilzköpfe aufweist, korrespondiert und somit auf diesem haftbar ist.

Bevorzugt ist vorgesehen, dass das erste Klettverschlussteil der Klebeplatte Schlaufen oder Pilzköpfe umfasst, insbesondere damit das erste Klettverschlussteil der Klebeplatte nicht an der Kleidung des Benutzers hängen bleiben kann. Allgemein kann ein mit Schlaufen versehenes Klettverschlussteil beispielsweise ein Velourband oder ein Flauschband sein.

In einer Ausgestaltung ist vorgesehen, dass die Klebeplatte das erste Klettverschlussteil und/oder das Band das zweite Klettverschlussteil aufweist. In einer Ausgestaltung ist vorgesehen, dass die Klebeplatte nur das erste Klettverschlussteil und/oder das Band nur das zweite Klettverschlussteil aufweist. Diese Ausgestaltung hat insbesondere den Vorteil, dass das Band nicht versehentlich auf sich selber haftet, wobei die bevorzugte justierende Wirkung verloren gehen könnte. Wenn das Band nur auf der Klebeplatte haftet, kann ein Zug nach posterior oder inferior auf die Trachealkanüle ausgeübt beziehungsweise reguliert werden.

Das erste Klettverschlussteil umfasst eine Klettverschlussbreite, die sich in einer bevorzugten Ausgestaltung über eine vollständige Klebeplattenbreite erstreckt. Weiterhin umfasst das erste Klettverschlussteil bevorzugt eine Klettverschlusslänge, die sich weiter bevorzugt über eine vollständige Klebeplattenlänge erstreckt. Erfindungsgemäß ist das erste Klettverschlussteil vollflächig auf der zweiten Oberfläche der Klebeplatte angeordnet.

Im Sinne der Erfindung ist eine Länge beziehungsweise eine Längsrichtung der Klebeplatte und/oder des Bandes definiert durch eine Längserstreckung des Bandes, welches von der Klebeplatte ausgeht. Insbesondere erstreckt sich die Länge des Befestigungsmittels beispielsweise im Wesentlichen nach medial in Richtung des Tracheostomas beziehungsweise der mit dem Befestigungsmittel befestigten Trachealkanüle.

In einer bevorzugten Ausgestaltung ist vorgesehen, dass die Klebeplatte breiter ist als das Band. In einer bevorzugten Ausgestaltung ist vorgesehen, dass das das erste Klettverschlussteil breiter ist als das zweite Klettverschlussteil. In einer Ausgestaltung ist vorgesehen, dass das erste Klettverschlussteil eine Klettverschlussbreite umfasst, die etwa doppelt bis etwa 15-mal, bevorzugt etwa 5-mal bis etwa 10-mal, weiter bevorzugt etwa 5-mal bis etwa 6-mal so groß ist wie eine Breite des Bandes. In einer Ausgestaltung ist vorgesehen, dass das erste Klettverschlussteil eine Klettverschlussbreite von etwa 15 mm bis etwa 50 mm umfasst, bevorzugt etwa 30 mm bis etwa 50 mm, weiter bevorzugt etwa 30 mm. In einer weiteren Ausgestaltung ist die Klettverschlusslänge die minimale Klettverschlusslänge. In einer weiteren Ausgestaltung ist vorgesehen, dass das erste Klettverschlussteil eine Klettverschlussbreite von etwa 20 mm bis etwa 50 mm umfasst, bevorzugt etwa 30 mm bis etwa 50 mm, weiter bevorzugt etwa 30 mm bis etwa 35 mm. In einer Ausgestaltung ist die Klettverschlussbreite die minimale Klettverschlussbreite. In einer Ausgestaltung ist die breiteste Stelle des ersten Klettverschlussteils etwa 30 mm bis etwa 40 mm, bevorzugt etwa 35 mm. In einer Ausgestaltung ist die schmalste Stelle des ersten Klettverschlussteils etwa 25 mm bis etwa 35 mm, bevorzugt etwa 30 mm.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen. Soweit verschiedene Wertebereiche, beispielsweise bevorzugte und weiter bevorzugte Wertebereiche, in der vorliegenden Erfindung angegeben sind, sind die Untergrenzen und die Obergrenzen der verschiedenen Wertebereiche miteinander kombinierbar.

In einer Ausgestaltung ist das erste Klettverschlussteil trapezförmig, quadratisch, rund oder oval. Bevorzugt weist das erste Klettverschlussteil abgerundete Ecken auf. Bevorzugt ist vorgesehen, dass eine breitere Seite, beispielsweise eines trapezförmigen ersten Klettverschlussteil der Klebeplatte, im Wesentlichen lateral anordenbar ist, wobei bevorzugt das Band sich im Wesentlichen in mediale Richtung erstreckt.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

Besonders bevorzugt ist die Klettverschlussbreite zumindest etwa doppelt so groß, bevorzugt etwa 3-mal bis etwa 10-mal, weiter bevorzugt etwa 4-mal bis etwa 7-mal, weiter bevorzugt etwa 6-mal so groß wie die Breite des Bandes, insbesondere einer Breite des zweiten Klettverschlussteils des Bandes.

Das an der Klebeplatte befestigbare Band ist bevorzugt durch ein Haltemittel, insbesondere eine Halteöse einer Trachealkanüle, fädelbar. Wenn das Band mit dem zweiten Klettverschlussteil auf das erste Klettverschlussteil der Klebeplatte gehaftet ist, formt das Band eine Schlaufe, mit der die Trachealkanüle zumindest einseitig haltbar ist. Vorteilhafterweise bietet das erste Klettverschlussteil der Klebeplatte mittels seiner Klettverschlussbreite eine Möglichkeit der Variation des Haftens des Bandes auf dem ersten Klettverschlussteils der Klebeplatte. Hierdurch ist vorteilhafterweise ermöglicht, dass auf die Trachealkanüle ein definierter Zug nach inferior oder posterior und/oder weiter bevorzugt ein definierter Zug nach lateral erfolgt. So kann die Trachealkanüle einfach ausgerichtet beziehungsweise feinjustiert werden, falls die Klebeplatte nicht die optimale Position auf der Haut hat und/oder eine Anpassung an eine Tragesituation erfolgen soll. Insbesondere ein trapezförmiges erstes Klettverschlussteil bietet den Vorteil, dass das Band leicht abgewinkelt nach superior oder leicht abgewinkelt nach inferior auf der Klebeplatte haftbar ist und gleichzeitig eine möglichst große Haftfläche Klettverschlussteile aufeinander gegeben ist, wobei insbesondere gleichzeitig in der Nähe des Tracheostomas, wo das Gewebe meist besonders empfindlich ist, eine möglichst kleine Fläche von der Klebeplatte bedeckt ist.

Werden im Rahmen der Beschreibung der Erfindung Richtungsangaben verwendet, so sind diese in Bezug auf den üblichen Gebrauch des Befestigungsmittels zu verstehen. Richtungsbezeichnungen werden wie in der Anatomie üblich verwendet. Unter dem Begriff "lateral" wird im Sinne der vorliegenden Erfindung in Bezug auf ein Merkmal der erfindungsgemäßen Vorrichtung eine Anordnung bzw. Verwendung desselben von einer Medianebene weg beziehungsweise zur Seite hin verstanden. Beispielsweise werden die Befestigungsmittel bevorzugt im Wesentlichen lateral, das heißt rechts und/oder links des Körpers in einer anatomischen Grundposition, des Tracheostomas befestigt. Unter dem Begriff "medial" wird im Sinne der vorliegenden Erfindung in Bezug auf ein Merkmal der erfindungsgemäßen Vorrichtung eine Anordnung beziehungsweise Verwendung desselben zu einer Medianebene hin beziehungsweise zur Mitte hin verstanden. Insbesondere sind lateral und medial entgegengesetzte Richtungsangaben. Unter superior ist vorzugsweise eine relative Richtungsangabe längs oder parallel der Körperlängsachse oberhalb eines Bezuges zu verstehen. Insbesondere ist unter superior "oben" in einer anatomischen Grundposition zu verstehen. Unter inferior ist vorzugsweise eine relative Richtungsangabe längs oder parallel der Körperlängsachse unterhalb eines Bezuges zu verstehen. Insbesondere ist unter inferior "unten" in einer anatomischen Grundposition zu verstehen. Beim Menschen ist die anatomische Grundposition aufrechter Stand, die Augen geradeaus, die Hände supiniert, das heißt die Handflächen nach vorn, und die Füße stehen parallel.

Die Klebeplatte umfasst eine Klebeplattenbreite. Weiterhin umfasst die Klebeplatte bevorzugt eine Klebeplattenlänge. In einer Ausgestaltung ist vorgesehen, dass die Klebeplatte eine Klebeplattenlänge von etwa 15 mm bis etwa 50 mm umfasst, bevorzugt etwa 30 mm bis etwa 50 mm, weiter bevorzugt etwa 30 mm. In einer weiteren Ausgestaltung ist die Klebeplattenlänge die minimale Klebeplattenlänge. In einer weiteren Ausgestaltung ist vorgesehen, dass die Klebeplatte eine Klebeplattenbreite von etwa 20 mm bis etwa 50 mm umfasst, bevorzugt etwa 30 mm bis etwa 50 mm, weiter bevorzugt etwa 30 mm bis etwa 35 mm. In einer Ausgestaltung ist die Klebeplattenbreite die minimale Klebeplattenbreite. In einer Ausgestaltung ist die breiteste Stelle der Klebeplatte etwa 30 mm bis etwa 40 mm, bevorzugt etwa 35 mm. In einer Ausgestaltung ist die schmalste Stelle der Klebeplatte etwa 25 mm bis etwa 35 mm, bevorzugt etwa 30 mm.

In einer Ausgestaltung ist die Klebeplatte trapezförmig, quadratisch, rund oder oval. Bevorzugt weist die Klebeplatte abgerundete Ecken auf. Bevorzugt weisen die abgerundeten Ecken einen Radius von etwa 4 mm bis etwa 15 mm, bevorzugt etwa 5 mm bis etwa 10 mm auf. Weiter bevorzugt sind insbesondere die Ecken auf der vom Band abgewandten Seite abgerundet. Weiter bevorzugt weisen die Ecken an der dem Band zugewandten Seite einen kleineren Radius als auf der vom Band abgewandten Seite. Beispielsweise umfassen die Ecken an der dem Band zugewandten Seite einen Radius von etwa 5 mm und die Ecken an der vom Band abgewandten Seite einen Radius von etwa 10 mm. Die abgerundeten Ecken haben den Vorteil, dass Reizungen der Haut durch winklige Kanten vermieden werden können. Vorteilhafterweise ist eine Klebeplatte mit abgerundeten Ecken komfortabler zu tragen. Bevorzugt ist vorgesehen, dass eine breitere Seite, beispielsweise einer trapezförmigen Klebeplatte, im Wesentlichen lateral anordenbar ist, wobei das Band sich im Wesentlichen in mediale Richtung erstreckt. Besonders bevorzugt ist die Klebeplattenbreite zumindest doppelt so groß, wie die Breite des Bandes.

In einer Ausgestaltung ist vorgesehen, dass die Klebeplatte eine Dicke von etwa 2 mm bis etwa 10 mm, bevorzugt etwa 5 mm aufweist. Insbesondere ist die Klebeplatte gepolstert. Eine Polsterung kann beispielsweise ein offenporiger oder ein geschlossenporiger Schaumstoff sein, der zwischen der ersten Oberfläche und der zweiten Oberfläche angeordnet ist. Offenporiger Schaumstoff ist vorteilhafterweise sehr weich und nachgiebig und insbesondere für starke Bewegungen geeignet. Geschlossenporiger Schaumstoff ist hygienisch, da die Oberfläche für die Einnistung von Keimen klein ist. Zudem bietet geschlossenporiger Schaumstoff festen Halt.

Das Band ist derart ausgestaltet, dass es in die Ösen marktüblicher Trachealkanülen fädelbar ist. Mittels des Bandes kann eine einfache Befestigung der Trachealkanüle vorgenommen werden. Vorteilhafterweise kann das Band insbesondere über den Trageverlauf schnell und einfach neu justiert, insbesondere mittels Anpassung eines Zuges und/oder einer Zugrichtung auf die Trachealkanüle, und auf die aktuellen Bedürfnisse des Benutzers angepasst werden. Hierzu ist das Band von dem ersten Klettverschlussteil der Klebeplatte mittels einer Scherbewegung lösbar. Durch Zug ein eine gewünschte Richtung ist die Trachealkanüle im Tracheostoma justierbar. Nach der Justierung ist das Band in der justierten Position auf dem ersten Klettverschlussteil der Klebeplatte wieder haftend aufbringbar.

In einer Ausgestaltung ist vorgesehen, dass sich das Band von der Klebeplatte weg erstreckt. Bevorzugt erstreckt sich das Band im Wesentlichen in einer Längsrichtung der Klebeplatte von diesem weg.

Das zweite Klettverschlussteil des Bandes umfasst in einer Ausgestaltung Widerhaken, Schlaufen, Rundköpfe oder Pilzköpfe, die mit dem ersten Klettverschlussteil der Klebeplatte zusammen wirken beziehungsweise korrespondieren. Bevorzugt umfasst das zweite Klettverschlussteil Wiederhaken und/oder Pilzköpfe. Das zweite Klettverschlussteil des Bandes ist auf dem ersten Klettverschlussteil der Klebeplatte haftbar.

In einer bevorzugten Ausgestaltung ist das zweite Klettverschlussteil nur einseitig auf dem Band aufgebracht. Insbesondere ist das zweite Klettverschlussteil nur auf der zweiten Seite des Bandes aufgebracht, die der zweiten Seite der Klebeplatte entspricht. Durch Umklappen des Bandes beziehungsweise Formen einer Schlaufe kann die zweite Seite des Bandes mit der zweiten Seite der Klebeplatte in Kontakt kommen und bevorzugt auf dieser haften.

Eine Bandlänge ist etwa 60 mm bis etwa 70 mm, bevorzugt etwa 68 mm. In einer Ausgestaltung ist vorgesehen, dass die Breite des Bandes etwa 2 mm bis etwa 15 mm, bevorzugt etwa 5 mm ist.

In einer Ausgestaltung ist vorgesehen, dass das Band einteilig mit der Klebeplatte verbunden ist. Unter einteilig im Sinne der Erfindung ist zu verstehen, dass das Band nicht zerstörungsfrei lösbar mit der Klebeplatte verbunden ist. Einteilige Befestigungsmittel können einstückig ausgestaltet sein, indem Band und Klebeplatte aus einem Stück bestehen. Weiterhin können einteilige Befestigungsmittel zweistückig ausgestaltet sein, bei dem Band und Klebeplatte zwei nicht zerstörungsfrei lösbar miteinander verbundene Stücke sind.

Insbesondere können Band und Klebeplatte bei einer zweistückigen Ausgestaltung aus unterschiedlichen Materialien sein. Bevorzugt können Band und Klebeplatte formschlüssig und/oder stoffschlüssig miteinander gefügt sein. In einer Ausgestaltung ist vorgesehen, dass das Band an die Klebeplatte genäht, geklebt, geschweißt und/oder genietet ist. Bevorzugt ist eine genähte, geklebte, geschweißte und/oder genietete Verbindung von Band und Klebeplatte eine einteilige Verbindung derselben. Eine einteilige, zweistückige Ausgestaltung von Band und Klebeplatte, bei der Band und Klebeplatte aus unterschiedlichen Materialien gefertigt sind, hat den Vorteil, dass die Klebeplatte beispielsweise weicher oder rigider ausgestaltet sein kann als das Band, um bevorzugt einen erhöhten Tragekomfort zu gewährleisten und gleichzeitig eine durch das Band feste Verbindung zur Trachealkanüle zu gewährleisten.

In einer weiteren Ausgestaltung ist vorgesehen, dass das Band mit der Klebeplatte einstückig ausgebildet ist. Eine einstückige Ausgestaltung, das heißt eine aus einem Stück hergestellte Klebeplatte mit Band, hat den Vorteil, dass die Herstellung weniger Schritte erfordert und somit günstiger ist, beispielsweise muss das Band nicht an die Klebeplatte genäht oder geschweißt werden. Zudem ist eine einstückige Ausgestaltung besonders haltbar. Ein Vorteil der einteiligen Verbindung ist, unabhängig davon ob diese einstückig oder zweistückig ausgestaltet ist, dass das Befestigungsmittel einen definierten sicheren Halt gibt. Durch die einseitige, feste beziehungsweise unlösbare Verbindung des Bandes an der Klebeplatte bei der Bildung einer Schlaufe des Bandes, wird vorteilhaft erreicht, dass das Band nicht so leicht verrutscht, beziehungsweise die mit dem Befestigungsmittel befestigte Trachealkanüle nicht die Position ändert, wenn der Benutzer sich im Alltag bewegt. Wenn das Band beispielsweise mit beiden Enden mittels eines Klettverschlusses auf der Klebeplatte angeordnet werden würde, um die Trachealkanüle zu fixieren, können sich beide Enden des Bandes leicht lockern. Dies führt dann eher zu einer merklichen Positionsänderung der Trachealkanüle im Vergleich zu einem Band, das einteilig mit der Klebeplatte verbunden ist.

In einer weiteren Ausgestaltung ist das Band zweiteilig mit der Klebeplatte verbunden. So ist in einer Ausgestaltung das Band beispielsweise mittels eines Knopfes oder eines Klettverschlussteils auf der Klebeplatte befestigbar. Beispielweise umfasst das Band beidseitig das zweite Klettverschlussteil. Das Band umfasst insbesondere ein erstes Ende und ein zweites Ende, wobei beide Enden auf der Klebeplatte befestigbar sind, um eine Schlaufe zu bilden, mittels der die Trachealkanüle fixierbar ist. Zur Justierung der Trachealkanüle ist das erste Ende und das zweite Ende mittig, an der gleichen Längsseite der Klebeplatte oder beliebig auf der Klebeplatte anbringbar. Insbesondere ist das erste Ende weiter medial als das zweite Ende befestigbar. Sind beide Enden beispielsweise an einer Längsseite der Klebeplatte befestigt, so ist ein definierter Zug zumindest in Richtung dieser Längsseite auf die Trachealkanüle ausgeübt.

In einer weiteren Ausgestaltung ist vorgesehen, dass das Band, beispielsweise zum Vernähen oder Vernieten des Bandes mit der Klebeplatte, etwa 2 mm bis etwa 10 mm, bevorzugt etwa 5 mm auf der Klebeplatte, bevorzugt auf der zweiten Oberfläche der Klebeplatte angeordnet ist.

Mit dem Band wird eine schnell herzustellende und leicht zu justierende Verbindung von Trachealkanüle zur Klebeplatte und hierüber zur Haut des Benutzers hergestellt.

Ein beispielhaftes Befestigungsmittel weist eine Klebeplatte und ein an diesem befestigtes Band auf. Das Band ist beispielsweise an die Klebeplatte auf einer zweiten Oberfläche angeordnet, die vollflächig ein erstes Klettverschlussteil aufweist. Das Band weist zumindest einseitig, bevorzugt nur einseitig, ein zweite Klettverschlussteil auf, das auf dem ersten Klettverschlussteil der Klebeplatte haftbar ist. Auf der ersten Oberfläche, die der zweiten Seite gegenüber liegt, ist beispielsweise die Klebeschicht angeordnet, auf der die abziehbare Schutzschicht aufgeklebt ist. Mittels einer Lasche, die beispielsweise mittels eines Teilschnittes von der übrigen Klebeplatte abgesetzt ist, kann die Schutzschicht komfortabel von der Klebeschicht, die auf der ersten Seite der Klebeplatte angeordnet ist, abgelöst werden. Der Teilschnitt durchtrennt bevorzugt ausgehend von der zweiten Oberfläche der Klebeplatte im Wesentlichen bis zur Klebeschicht aber zumindest nicht die Schutzschicht.

Das Band erstreckt sich in Längsrichtung der Klebeplatte von dieser weg. Das Band umfasst beispielsweise eine Bandlänge von etwa 65 mm bis etwa 70 mm und beispielsweise eine Breite des Bandes von etwa 5 mm. Das Band ragt etwa 5 mm auf die zweite Oberfläche der Klebeplatte und ist mit dieser beispielsweise vernäht. Die Klebeplatte weist beispielsweise eine Klebeplattenlänge 22 von etwa 30 mm und beispielsweise eine maximale Klebeplattenbreite von etwa 30 mm bis etwa 35 mm auf beziehungsweise ist etwa fünf bis etwa sieben Mal breiter als das Band. Insbesondere bei abgetrennter Lasche ist die Klebeplatte im Wesentlichen trapezförmig mit abgerundeten Kanten. Auf der zweiten Oberfläche der Klebeplatte ist das erste Klettverschlussteil vollflächig auf der Klebeplatte aufgebracht, so dass eine Klettverschlussbreite der Klebeplattenbreite und eine Klettverschlusslänge der Klebeplattenlänge entspricht. Die Klebeplatte weist beispielsweise eine Klebeplattendicke von etwa 5 mm auf.

Vorteilhaft an allen Ausgestaltungen des Befestigungsmittels ist insbesondere, dass mittels der Befestigungsmittel die Trachealkanüle derart im Tracheostoma fixiert wird, dass die Fixierung an unterschiedliche Tragesituationen anpassbar ist. Beispielsweise können Tragesituationen das Schauen eines Kinofilms, bei der der Kopf des Benutzers längere Zeit nach oben geneigt ist, oder feinmechanische Arbeiten, bei denen der Kopf längere Zeit nach unten geneigt ist, sein.

Beispielhafte Aufzählungen sind im Sinne der Erfindung als nicht abschließend anzusehen, sondern können im Rahmen des allgemeinen Fachwissens ergänzt werden. In einer beispielhaften Ausgestaltung ist vorgesehen, dass das Befestigungsmittel eine Klebeplatte und ein an diesem befestigtes Band aufweist. Das Band ist beispielsweise an der Klebeplatte auf einer zweiten Oberfläche angeordnet, die vollflächig ein erstes Klettverschlussteil aufweist. Das Band weist einseitig ein zweites Klettverschlussteil auf, die auf das erste Klettverschlussteil der Klebeplatte haftbar ist. Mittels einer Lasche, die mittels eines Teilschnittes von der übrigen Klebeplatte abgesetzt ist, kann eine Schutzschicht komfortabel von einer Klebeschicht abgelöst werden. Der Teilschnitt durchtrennt bevorzugt ausgehend von der zweiten Oberfläche die Klebeplatte im Wesentlichen bis zur Klebeschicht aber zumindest nicht die Schutzschicht.

Das Band erstreckt sich beispielhaft in Längsrichtung der Klebeplatte. Das Band umfasst eine Bandlänge von etwa 65 mm bis etwa 70 mm und eine Breite des Bandes von beispielsweise etwa 5 mm. Das Band ragt etwa 5 mm auf die zweite Oberfläche der Klebeplatte und ist mit diesem beispielsweise vernäht. Die Klebeplatte weist eine Klebeplattenlänge von etwa 30 mm und eine maximale Klebeplattenbreite von etwa 30 mm bis etwa 35 mm auf beziehungsweise ist etwa fünf bis etwa sieben Mal breiter als das Band. Bei abgetrennter Lasche ist die Klebeplatte im Wesentlichen trapezförmig mit abgerundeten Kanten. Auf der zweiten Oberfläche der Klebeplatte ist das erste Klettverschlussteil vollflächig auf der Klebeplatte aufgebracht, so dass eine Klettverschlussbreite der Klebeplattenbreite und eine Klettverschlusslänge einer Klebeplattenlänge entspricht. Die Klebeplatte weist beispielsweise eine Klebeplattendicke von etwa 5 mm auf. Auf der ersten Oberfläche, die der zweiten Seite gegenüber liegt, ist die Klebeschicht angeordnet, auf der die abziehbare Schutzschicht 16 aufgeklebt ist

Weiterhin wird ein Set umfassend zumindest zwei Klebeplatten mit jeweils einer ersten Oberfläche und einer zweiten Oberfläche und mindestens zwei mit den Klebeplatten verbindbare Bänder zur Verbindung mit einem Haltemittel einer Trachealkanüle vorgeschlagen. Jeweils die erste Oberfläche der Klebeplatten umfasst eine Klebeschicht zum Aufkleben auf eine Hautoberfläche und jeweils die zweite Oberfläche umfasst ein erstes Klettverschlussteil wobei sich das erste Klettverschlussteil vollflächig über die erste Oberfläche der Klebeplatte erstreckt,. Die Bänder umfassen jeweils zumindest einseitig ein zweites Klettverschlussteil, das mit dem ersten Klettverschlussteil zumindest einer der Klebeplatten korrespondiert, wobei mittels jeweils eines Bandes und einer Klebeplatte ein Befestigungsmittel bildbar ist. Mittels des Sets kann eine Befestigung einer Trachealkanüle beidseitig an der Haut des Benutzers erfolgen.

Das Set weist bevorzugt jeweils etwa 2 bis etwa 12, weiter bevorzugt etwa 2 bis etwa 6, weiter bevorzugt etwa 2 bis etwa 4 Klebeplatten und Bänder auf.

In einer Ausgestaltung ist vorgesehen, dass mittels jeweils eines Bandes und einer Klebeplatte ein Befestigungsmittel bildbar ist. In einer weiteren Ausgestaltung ist vorgesehen, dass das Set zumindest jeweils eine Klebeplatte und ein Band, die zu einem Befestigungsmittel verbunden sind, umfasst. In einer weiteren Ausgestaltung ist vorgesehen, dass das Set zumindest jeweils eine Klebeplatte und ein Band, die zweistückig, bevorzugt dabei einteilig, oder einstückig zu einem Befestigungsmittel verbunden sind, umfasst. Bevorzugt ist das Band auf das Befestigungsmittel mittels eines Klettverschlussteils aufbingbar. Bevorzugt weist das Band hierzu beidseitig das zweite Klettverschlussteil auf. Vorteilhaft kann somit das Band frei auf der Klebeplatte positioniert werden, so dass insbesondere kein Verdrillen des Bandes erfolgt, wenn mittels des Bandes ein Zug nach inferior oder posterior erreicht werden soll. Beispielswiese kann das Band mit einem ersten Ende mittig oder an einer der Längsseiten der Klebeplatte und mit einem zweite Ende ebenfalls etwa mittig oder an der Längsseite der Klebeplatte befestigt werden, wobei bevorzugt das zweie Ende weiter lateral befestigt wird als das erste Ende.

In einer weiteren Ausgestaltung ist vorgesehen, dass das Band und die Klebeplatte zweistückig ausgestaltet sind, wobei Band und Klebeplatte zerstörungsfrei voneinander lösbar sind. In einer weiteren Ausgestaltung ist vorgesehen, dass das Band mittels eines Knopfes, beispielsweise eines Druccknopfes, oder eine Klettverschlusses auf die Klebeplatte aufbringbar ist.

In einer weiteren Ausgestaltung ist vorgesehen, dass das Set zumindest zwei montierte Befestigungsmittel umfasst. Beispielsweise umfasst das Set zumindest zwei, bevorzugt etwa 2 bis etwa 12, weiter bevorzugt etwa 2 bis etwa 6, weiter bevorzugt etwa 2 bis etwa 4 montierte Befestigungsmittel auf. Beispielsweise sind Klebeplatten und Bänder der jeweiligen Befestigungsmittel einteilig oder einstückig miteinander verbunden.

In einer beispielhaften Ausgestaltung eines erfindungsgemäßen Sets umfasst dieses zwei Klebeplatten sowie zwei Bänder, wobei jeweils eine Klebeplatte und ein Band miteinander verbunden sind und jeweils ein Befestigungsmittel bilden. Die zwei Befestigungsmittel sind an eine Trachealkanüle anbringbar. Die Bänder sind durch Haltemittel, die beispielsweise als Halteösen ausgestaltet sind, der Trachealkanüle führbar und danach auf die ersten Klettverschlussteile der jeweiligen Klebeplatten befestigt. Die Bänder bilden so eine Schlaufe, mittels der die Klebeplatten an der Trachealkanüle befestigt sind.

Die Trachealkanüle wird dann bei leichter Rückneigung des Kopfes eines Benutzers und während eines Einatmens in das Tracheostoma eingeführt. Die Befestigungsmittel stören die Einführung nicht. Daraufhin kann die Schutzschicht vom Klebeplatte gelöst werden.

Die Klebeplatten sind beispielsweise manuell auf einer Haut des Benutzers festdrückbar. Die Klebeplatten werden bei dieser Form der Anbringung relativ zum Tracheostoma komfortabel angebracht. Ein unangenehmer Zug auf die Trachealkanüle erfolgt schon deshalb nicht, da die an die Trachealkanüle vormontierten Klebeplatten die Klebeposition vorgeben, wobei der Benutzer Korrekturen vornehmen kann, in dem er beispielsweise die Klebeplatten vor dem Festdrücken beispielsweise leicht nach posterior oder inferior führt. Eine Feineinstellung des Zuges der Bänder auf die Trachealkanüle kann auch noch nach dem Aufkleben der Klebeplatten durch Lösen der Bänder von den ersten Klettverschlussteilen und Wiederanbringen auf den ersten Klettverschlussteilen in einer anderen Position erfolgen. Auch kann beispielsweise abhängig von einer Tätigkeit des Benutzers die Feineinstellung mittels der auf den ersten Klettverschlussteilen haftenden Bänder erfolgen

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: eine perspektivische Ansicht eines Befestigungsmittels;
- Fig. 2: eine Aufsicht auf das Befestigungsmittel aus Fig. 1;
- Fig. 3: eine Seitenansicht des Befestigungsmittels aus Fig. 1;
- Fig. 4: eine Trachealkanüle mit einem Set umfassend zwei Befestigungsmitteln;
- Fig. 5: die Trachealkanüle aus Fig. 4, beim Einführen in ein Tracheostoma;
- Fig. 6: eine Befestigung der Befestigungsmittel auf der Haut eines Benutzers; und
- Fig. 7: ein Ablösen des Befestigungsmittels von einer Haut des Benutzers

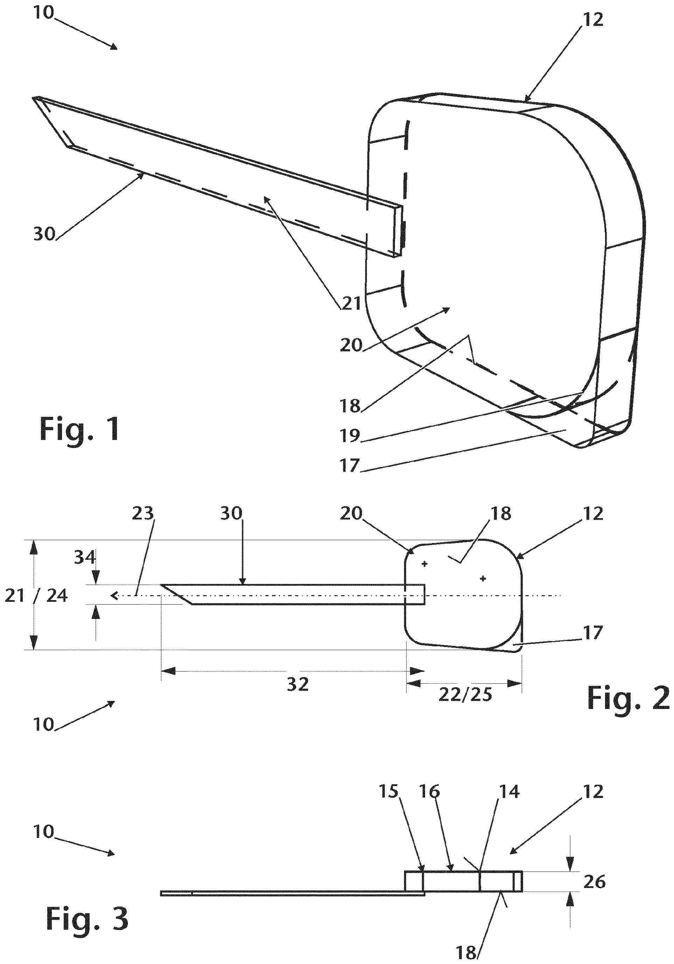

Fig. 1 zeigt eine perspektivische Ansicht eines Befestigungsmittels 10 für Trachealkanülen. Das Befestigungsmittel 10 weist eine Klebeplatte 12 und ein an diesem befestigtes Band 30 auf. Das Band 30 ist beispielsweise an der Klebeplatte 12 auf einer zweiten Oberfläche 18 angeordnet, die vollflächig ein erstes Klettverschlussteil 20 aufweist. Das Band 30 weist einseitig ein zweites Klettverschlussteil 21 auf, die auf das erste Klettverschlussteil 20 der Klebeplatte haftbar ist.

Mittels einer Lasche 17, die mittels eines Teilschnittes 19 von der übrigen Klebeplatte 12 abgesetzt ist, kann eine in Fig. 3 bezeichnete Schutzschicht 16 komfortabel von einer in Fig. 3 bezeichneten Klebeschicht 15 abgelöst werden. Der Teilschnitt 19 durchtrennt bevorzugt ausgehend von der zweiten Oberfläche 18 die Klebeplatte im Wesentlichen bis zur Klebeschicht aber zumindest nicht die Schutzschicht 16.

Fig. 2 zeigt eine Aufsicht auf das Befestigungsmittel 10. Das Band 30 erstreckt sich in Längsrichtung 23 der Klebeplatte 12. Das Band 30 umfasst eine Bandlänge 32 von etwa 65 mm bis etwa 70 mm und eine Breite des Bandes 34 von etwa 5 mm. Das Band 30 ragt etwa 5 mm auf die zweite Oberfläche 18 der Klebeplatte 12 und ist mit diesem beispielsweise vernäht. Die Klebeplatte 12 weist eine Klebeplattenlänge 22 von etwa 30 mm und eine maximale Klebeplattenbreite 24 von etwa 30 mm bis etwa 35 mm auf beziehungsweise ist etwa fünf bis etwa sieben Mal breiter als das Band. Bei abgetrennter Lasche 17 ist die Klebeplatte 12 im Wesentlichen trapezförmig mit abgerundeten Kanten. Auf der zweiten Oberfläche 18 der Klebeplatte 12 ist das erste Klettverschlussteil 20 vollflächig auf der Klebeplatte aufgebracht, so dass eine Klettverschlussbreite 21 der Klebeplattenbreite 24 und eine Klettverschlusslänge 25 einer Klebeplattenlänge 22 entspricht.

Fig. 3 zeigt eine Ansicht des Befestigungsmittels 10 in einer Seitenansicht. Die Klebeplatte 12 weist eine Klebeplattendicke 26 von etwa 5 mm auf. Auf der ersten Oberfläche 14, die der zweiten Seite 18 gegenüber liegt, ist die Klebeschicht 15 angeordnet, auf der die abziehbare Schutzschicht 16 aufgeklebt ist.

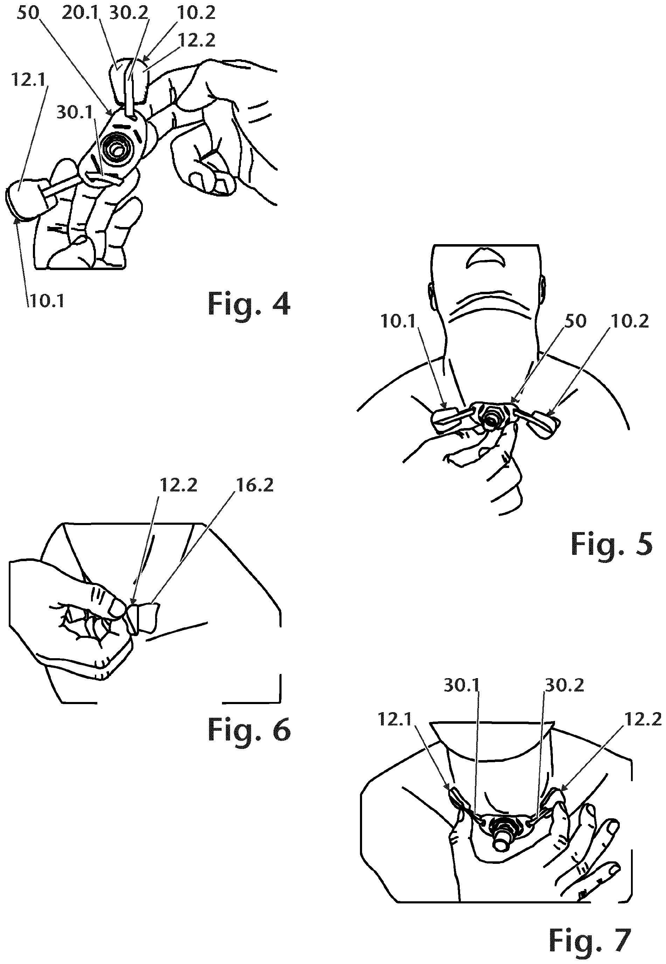

Fig. 4 zeigt eine Trachealkanüle 50 und ein Set umfassend zwei Klebeplatten 12.1 und 12.2 sowie zwei Bänder 30.1 und 30.2, wobei jeweils eine Klebeplatte 12.1, 12.2 und ein Band 30.1, 30.2 miteinander verbunden sind und jeweils ein Befestigungsmittel 10.1 und 10.2 bilden. Die zwei Befestigungsmittel 10.1 und 10.2 sind an der Trachealkanüle 50 anbringbar. Die Bänder 30.1 und 30.2 werden durch Haltemittel, die als Halteösen ausgestaltet und die der Übersichtlichkeit halber nicht in Fig. 4 bezeichnet sind, der Trachealkanüle 50 geführt und werden anschließend auf die ersten Klettverschlussteile 20.1 und 20.2 der jeweiligen Klebeplatten 12.1 und 12.2 angeordnet beziehungsweise befestigt. Die Bänder 30.1 und 30.2 bilden eine Schlaufe, mittels der die Klebeplatten 12.1 und 12.2 an der Trachealkanüle 50 befestigt sind.

Fig. 5 zeigt die Einführung der Trachealkanüle 50 in ein hier nicht dargestelltes Tracheostoma, wobei insbesondere bei leichter Rückneigung des Kopfes eines Benutzers und während eines Einatmens das Tracheostoma eingeführt wird. Die Befestigungsmittel 10.1 und 10.2 stören die Einführung nicht. Fig. 6 zeigt das Lösen der Schutzschicht 16.2 vom Klebeplatte 12.2.

Fig. 7 zeigt das manuelle Festdrücken der Klebeplatten 12.1 und 12.2 auf einer Haut des Benutzers. Die Klebeplatten 12.1 und 12.2 werden bei dieser Form der Anbringung relativ zum Tracheostoma komfortabel angebracht. Ein unangenehmer Zug erfolgt nicht, da die an die Trachealkanüle 50 vormontierten Klebeplatten 12.1 und 12.2 die Klebeposition vorgeben, wobei der Benutzer Korrekturen vornehmen kann, in dem er die Klebeplatten 12.1 und 12.2 vor dem Festdrücken beispielsweise leicht nach posterior oder inferior führt. Eine Feineinstellung des Zuges der Bänder auf die Trachealkanüle kann auch noch nach dem Aufkleben der Klebeplatten 12.1 und 12.2 durch Lösen der Bänder 30.1 und 30.2 von der in Fig. 4 bezeichneten ersten Klettverschlussteilen 20.1 und 20.2 und Wiederanbringen auf den ersten Klettverschlussteilen 20.1 und 20.2 in einer anderen Position erfolgen. Auch kann beispielsweise abhängig von einer Tätigkeit des Benutzers die Feineinstellung mittels der auf den ersten Klettverschlussteilen 20.1 und 20.2 haftenden Bänder 30.1 und 30.2 erfolgen.

## Patentansprüche

1. Befestigungsmittel (10) für Trachealkanülen umfassend eine Klebeplatte (12) mit einer ersten Oberfläche (14) und einer zweiten Oberfläche (18) und mindestens ein mit der Klebeplatte (12) verbundenes Band (30) zur Verbindung mit einem Haltemittel (50) einer Trachealkanüle, wobei die erste Oberfläche (14) eine Klebeschicht (15) zum Aufkleben auf eine Hautoberfläche und die zweite Oberfläche (18) ein erstes Klettverschlussteil (20) umfasst, wobei das Band (30) zumindest einseitig ein zweites Klettverschlussteil (21) umfasst, das mit dem ersten Klettverschlussteil (20) der Klebeplatte (12) korrespondiert, **dadurch gekennzeichnet, dass** sich das erste Klettverschlussteil (20) vollflächig über die erste Oberfläche (14) der Klebeplatte (12) erstreckt.

2. Befestigungsmittel (10) gemäß Anspruch 1, **dadurch gekennzeichnet, dass** die Klebeplatte (12) das erste Klettverschlussteil (20) und das Band (30) das zweite Klettverschlussteil (21) aufweist.

3. Befestigungsmittel (10) gemäß einem oder mehreren der vorhergehenden Ansprüche, **dadurch gekennzeichnet, dass** das erste Klettverschlussteil (20) eine Klettschichtbreite (21) umfasst, die etwa doppelt bis etwa 15-mal so groß ist wie eine Breite (34) des Bandes (30).

4. Befestigungsmittel (10) gemäß einem oder mehreren der vorhergehenden Ansprüche, **dadurch gekennzeichnet, dass** die Klebeplatte (12) eine Plattenlänge (22) von etwa 20 mm bis etwa 50 mm umfasst.

5. Befestigungsmittel (10) gemäß einem oder mehreren der vorhergehenden Ansprüche, **dadurch gekennzeichnet, dass** die Klebeplatte (12) eine Plattenbreite (24) von etwa 15 mm bis etwa 50 mm umfasst.

6. Befestigungsmittel (10) gemäß einem oder mehreren der vorhergehenden Ansprüche, **dadurch gekennzeichnet, dass** sich das Band (30) von der Klebeplatte (12) weg erstreckt.

7. Befestigungsmittel (10) gemäß einem oder mehreren der vorhergehenden Ansprüche, **dadurch gekennzeichnet, dass** die Breite (34) des Bandes (30) etwa 5 mm bis etwa 15 mm ist.

8. Befestigungsmittel (10) gemäß einem oder mehreren der vorhergehenden Ansprüche, **dadurch gekennzeichnet, dass** das Band (30) einteilig mit der Klebeplatte (12) verbunden ist.

9. Set umfassend zumindest zwei Klebeplatten (12.1, 12.2) mit jeweils einer ersten Oberfläche (14) und einer zweiten Oberfläche (18) und mindestens zwei mit den Klebeplatten (12.1, 12.2) verbindbare Bänder (30.1, 30.2), diese zur Verbindung mit einem Haltemittel einer Trachealkanüle (50), wobei jeweils die erste Oberfläche (14) der Klebeplatten (12.1, 12.2) eine Klebeschicht (15) zum Aufkleben auf eine Hautoberfläche und jeweils die zweite Oberfläche (16) ein erstes Klettverschlussteil (20.1, 20.2) umfasst, wobei sich das erste Klettverschlussteil (20) vollflächig über die erste Oberfläche der Klebeplatte (12) erstreckt, wobei die Bänder (30.1, 30.2) jeweils zumindest einseitig ein zweites Klettverschlussteil (21) umfassen, das mit dem ersten Klettverschlussteil (20.1, 20.2) zumindest einer der Klebeplatten (12.1, 12.2) korrespondiert, wobei mittels jeweils eines Bandes und einer Klebeplatte ein Befestigungsmittel (10.1, 10.2) gemäß einem oder mehreren der Ansprüche 1 bis 8 bildbar ist.

10. Set gemäß Anspruch 9, **dadurch gekennzeichnet, dass** es zumindest jeweils eine Klebeplatte (12.1, 12.2) und ein Band (30.1, 30.2), die zu einem Befestigungsmittel (10.1, 10.2) nach einem oder mehreren der Ansprüche 1 bis 8 verbunden sind, umfasst.

## Claims

1. Fastening means (10) for tracheal cannulae comprising an adhesive plate (12) having a first surface (14) and a second surface (18) and at least one tape (30) connected to the adhesive plate (12) for connection to a holding means (50) of a tracheal cannula, the first surface (14) comprising an adhesive layer (15) for adhering to a skin surface and the second surface (18) comprising a first hook-and-loop fastener part (20) wherein the tape (30) comprises, at least on one side, a second hook-and-loop fastener part (21) corresponding to the first hook-and-loop fastener part (20) of the adhesive plate (12), **characterized in that** the first hook-and-loop fastener part (20) extends over the entire surface of the first surface (14) of the adhesive plate (12).

2. Fastening means (10) according to claim 1, **characterized in that** the adhesive plate (12) has the first hook-and-loop fastener part (20) and the tape (30) has the second hook-and-loop fastener part (21).

3. Fastening means (10) according to one or more of the preceding claims, **characterized in that** the first hook and loop fastener part (20) comprises a hook and loop layer width (21) that is about twice to about 15 times as large as a width (34) of the tape (30).

4. Fastening means (10) according to one or more of the preceding claims, **characterized in that** the adhesive plate (12) comprises a plate length (22) of from about 20 mm to about 50 mm.

5. Fastening means (10) according to one or more of the preceding claims, **characterized in that** the adhesive plate (12) comprises a plate width (24) of from about 15 mm to about 50 mm.

6. Fastening means (10) according to one or more of the preceding claims, **characterized in that** the tape (30) extends away from the adhesive plate (12).

7. Fastening means (10) according to one or more of the preceding claims, **characterized in that** the width (34) of the tape (30) is about 5 mm to about 15 mm.

8. Fastening means (10) according to one or more of the preceding claims, **characterized in that** the tape (30) is integrally connected to the adhesive plate (12).

9. Set comprising at least two adhesive plates (12.1, 12.2), each having a first surface (14) and a second surface (18), and at least two tapes (30.1, 30.2) connectable to the adhesive plates (12.1, 12.2), these for connection to a holding means of a tracheal cannula (50), wherein in each case the first surface (14) of the adhesive plates (12.1, 12.2) comprises an adhesive layer (15) for adhering to a skin surface and in each case the second surface (16) comprises a first hook-and-loop fastener part (20.1, 20.2), wherein the first hook-and-loop fastener part (20) extends over the entire surface of the first surface of the adhesive plate (12), wherein the tapes (30.1, 30.2) each comprise, at least on one side, a second hook-and-loop fastener part (21) which corresponds with the first hook-and-loop fastener part (20. 1, 20.2) of at least one of the adhesive plates (12.1, 12.2), it being possible to form a fastening means (10.1, 10.2) according to one or more of claims 1 to 8 by means of in each case one tape and one adhesive plate.

10. Set according to claim 9, **characterized in that** it comprises at least one each of an adhesive plate (12.1, 12.2) and a tape (30.1, 30.2) joined to form a fastening means (10.1, 10.2) according to one or more of claims 1 to 8.

## Revendications

1. Moyen de fixation (10) de canules de trachéotomie comprenant une plaque adhésive (12) avec une première surface (14) et une deuxième surface (18) et au moins une bande (30) reliée à la plaque collante (12) pour la relier à un moyen de retenue (50) d'une canule de trachéotomie, la première surface (14) comprenant une couche adhésive (15) pour la coller sur une surface de peau et la deuxième surface (18) un premier élément auto-agrippant (20) et la bande (30) comprenant au moins d'un côté un deuxième élément auto-agrippant (21) qui correspond avec le premier élément auto-agrippant (20) de la plaque adhésive (12), **caractérisé en ce que** le premier élément auto-agrippant (20) s'étend sur toute la première surface (14) de la plaque adhésive (12).

2. Moyen de fixation (10) selon la revendication 1, **caractérisé en ce que** la plaque adhésive (12) présente le premier élément auto-agrippant (20) et la bande (30) le deuxième élément auto-agrippant (21).

3. Moyen de fixation (10) selon une ou plusieurs des revendications précédentes, **caractérisé en ce que** le premier élément auto-agrippant (20) comporte une largeur de couche auto-agrippante qui est d'environ deux fois à environ quinze fois plus grande qu'une largeur (34) de la bande (30).

4. Moyen de fixation (10) selon une ou plusieurs des revendications précédentes, **caractérisé en ce que** la plaque adhésive (12) présente une langueur de plaque (22) d'environ 20 mm à environ 50 mm.

5. Moyen de fixation (10) selon une ou plusieurs des revendications précédentes, **caractérisé en ce que** la plaque adhésive (12) présente une largeur de plaque (24) d'environ 15 mm à environ 50 mm.

6. Moyen de fixation (10) selon une ou plusieurs des revendications précédentes, **caractérisé en ce que** la bande (30) s'étend à partir de la plaque adhésive (12).

7. Moyen de fixation (10) selon une ou plusieurs des revendications précédentes, **caractérisé en ce que** la largeur (34) de la bande (30) est d'environ 5 mm à environ 15 mm.

8. Moyen de fixation (10) selon une ou plusieurs des revendications précédentes, **caractérisé en ce que** la bande (30) est reliée d'une seule pièce avec la plaque adhésive (12).

9. Set comprenant au moins deux plaques adhésives (12.1, 12.2) avec chacune une première surface (14) et une deuxième surface (18) et au moins deux bandes (30.1, 30.2) pouvant être reliées avec les plaques adhésives (12.1, 12.2), celles-ci servant à les relier avec un moyen de retenue d'une canule de trachéotomie (50), la première surface (14) de chacune des plaques adhésives (12.1, 12.2) comportant une couche adhésive (15) pour la coller sur une surface de peau et la deuxième surface (16) comportant un premier élément auto-agrippant (20,1, 20.2), le premier élément auto-agrippant (20) s'étendant sur la totalité de la première surface de la plaque adhésive (12), les bandes (30.1, 30.2) comportant chacune au moins d'un côté un deuxième élément auto-agrippant (21) qui correspond avec le premier élément auto-agrippant (20.1, 20.2) d'au moins une des plaques adhésives (12.1, 12.2) et un moyen de fixation (10.1, 10.2) selon une ou plusieurs des revendications 1 à 8 pouvant être réalisé au moyen d'une bande et d'une plaque adhésive.

10. Set selon la revendication 9, **caractérisé en ce qu'**il comprend au moins une plaque adhésive (12.1, 12.2) et une bande (30.1, 30.2) qui sont reliées à un moyen de fixation (10.1, 10.2) selon une ou plusieurs des revendications 1 à 8.
